# EUROPEAN PATENT APPLICATION

(11) **EP 3 770 606 A1**
(43) Date of publication of application: **27.01.2021**
(21) Application number: 19770838.1
(22) Date of filing: 18.03.2019
(51) Int. Cl.: G01N 33/68

(54) **METHOD FOR JUDGING PSYCHIATRIC DISORDER**

(30) Priority: 19.03.2018 JP 2018051017
(71) Applicant: FUJIFILM Wako Pure Chemical Corporation, Osaka 540-8605 (JP)
(72) Inventor: SAITO, Kuniaki, Toyoake-shi Aichi 470-1192 (JP); YAMAMOTO, Yasuko, Toyoake-shi Aichi 470-1192 (JP)
(74) Representative: Winter, Brandl, Fürniss, Hübner, Röss, Kaiser, Polte - Partnerschaft mbB
(86) International application number: PCT/JP2019/011091
(87) International publication number: WO 2019/181830

(57) **Abstract**

An object of the present invention is to provide a method for determining a psychiatric disorder such as depressive disorder, bipolar disorder, schizophrenia, or dementia with high reliability (accuracy and precision).

The present invention relates to a method for determining a psychiatric disorder, a marker for determining a psychiatric disorder, and the like.

## Description

### Technical Field

The present invention relates to a method for determining a psychiatric disorder such as depressive disorder, bipolar disorder, schizophrenia, or dementia.

### Background Art

In modern society, a psychiatric disorder such as depressive disorder, bipolar disorder, schizophrenia, or dementia has become a social problem. According to a survey by the Ministry of Health, Labor and Welfare in 2011, 3.2 million people suffer from psychiatric disorders. In addition, since a psychiatric disorder is common in a so-called productive age group between the ages of 15 to 65, the impact on society such as economic losses due to a decrease in labor force is immeasurable.

Particularly, depressive disorder characterized by symptoms such as depression, decreased mental activity, loss of appetite, and insomnia accounts for a high proportion of the psychiatric disorder. In addition, in recent years, depressive disorder has become a major social problem such as being a main cause of suicide.

However, since the diagnosis of a psychiatric disorder is currently made based on the subjective information of a patient obtained from an interview, it is difficult to make a diagnosis with high reliability (accuracy and precision).

Therefore, it has been desired to develop a method for diagnosing a psychiatric disorder using an objective index (such as a marker) with high reliability (accuracy and precision).

Various methods for diagnosing psychiatric disorders using the objective index (such as a marker) have been reported so far. For example, there are methods based on detecting markers using tryptophan and its metabolites in blood as the markers (Patent Document 1 and Non-Patent Documents 1 and 2).

However, none of these methods has been put to practical use.

### Citation List

### Patent Literature

[Patent Document 1] JP2008-537111A

### Non-Patent Literature

[Non-Patent Document 1] Darlington et al. On the Biological Importance of the 3-hydroxyanthranilic Acid: Anthranilic Acid Ratio. International Journal of Tryptophan Research 2010: 3 51-59
[Non-Patent Document 2] Gabbay et al. The possible role of the kynurenine pathway in adolescent depression with melancholic features. Journal of Child Psychology and Psychiatry 2010: 51 (8) 935-943

### Summary of Invention

### Technical Problem

An object of the present invention is to provide a method for determining a psychiatric disorder such as depressive disorder, bipolar disorder, schizophrenia, or dementia with high reliability (accuracy and precision).

### Solution to Problem

The present invention has been made to solve the problems, and has the following configurations.
[1] A method for determining a psychiatric disorder comprising the following steps (1) to (3):
   (1) step 1 of measuring an amount (A) of anthranilic acid in a sample, and an amount (B) of tryptophan, kynurenine, or 3-hydroxyanthranilic acid in the sample;
   (2) step 2 of (i) calculating a ratio ((A)/(B)) of the amount (A) of anthranilic acid to the amount (B) of tryptophan, kynurenine, or 3-hydroxyanthranilic acid, or (ii) calculating a ratio ((B)/(A)) of the amount (B) of tryptophan, kynurenine, or 3-hydroxyanthranilic acid to the amount (A) of anthranilic acid; and
   (3) step 3 of determining a psychiatric disorder based on a calculation result of the step 2.
[2] The determining method according to [1], in which the step 2 is a step of (i) calculating a ratio ((A)/(B)) of the amount (A) of anthranilic acid to the amount (B) of tryptophan, kynurenine, or 3-hydroxyanthranilic acid.
[3] The determining method according to [1] or [2], in which the step 2 is a step of calculating a ratio of the amount of anthranilic acid to the amount of kynurenine or 3-hydroxyanthranilic acid.
[4] The determining method according to any one of [1] to [3], in which the psychiatric disorder is depressive disorder, bipolar disorder, schizophrenia, or dementia.
[5] The determining method according to any one of [1] to [4], in which the psychiatric disorder is depressive disorder.
[6] The determining method according to any one of [1] to [5], in which the sample is serum, plasma, whole blood, urine, or cerebrospinal fluid.
[7] A method for obtaining data for determining a psychiatric disorder, the method comprising the following steps (1) and (2):
   (1) step 1 of measuring an amount (A) of anthranilic acid in a sample, and an amount (B) of tryptophan, kynurenine, or 3-hydroxyanthranilic acid in the sample; and
   (2) step 2 of (i) calculating a ratio ((A)/(B)) of the amount (A) of anthranilic acid to the amount (B) of tryptophan, kynurenine, or 3-hydroxyanthranilic acid, or (ii) calculating a ratio ((B)/(A)) of the amount (B) of tryptophan, kynurenine, or 3-hydroxyanthranilic acid to the amount (A) of anthranilic acid.
[8] The method for obtaining data according to [7], in which the step 2 is a step of (i) calculating a ratio ((B)/(A)) of the amount (A) of anthranilic acid to the amount (B) of tryptophan, kynurenine, or 3-hydroxyanthranilic acid.
[9] The method for obtaining data according to [7] or [8], in which the step 2 is a step of calculating a ratio of the amount of anthranilic acid to the amount of kynurenine or 3-hydroxyanthranilic acid.
[10] The method for obtaining data according to any one of [7] to [9], in which the psychiatric disorder is depressive disorder, bipolar disorder, schizophrenia, or dementia.
[11] The method for obtaining data according to any one of [7] to [10], in which the psychiatric disorder is depressive disorder.
[12] The method for obtaining data according to any one of [7] to [11], in which the sample is serum, plasma, whole blood, urine, or cerebrospinal fluid.
[13] A marker for determining a psychiatric disorder comprising: anthranilic acid; and tryptophan, kynurenine, or 3-hydroxyanthranilic acid.

### Advantageous Effects of Invention

According to the method for determining a psychiatric disorder and a marker for determining a psychiatric disorder of the present invention, it is possible to determining (diagnose and inspect) a psychiatric disorder such as depressive disorder, bipolar disorder, schizophrenia, or dementia with high reliability (accuracy and precision). In addition, according to the method for obtaining data for determining a psychiatric disorder according to the present invention, it is possible to obtain data for accurately performing the determination of a psychiatric disorder such as depressive disorder, bipolar disorder, schizophrenia, or dementia.

### Description of Embodiments

### Method for Determining Psychiatric Disorder of the Present Invention

A method for determining a psychiatric disorder according to the present invention (hereinafter, sometimes abbreviated as a determining method of the present invention) comprises
(1) step 1 of measuring an amount (A) of anthranilic acid in a sample, and an amount (B) of tryptophan, kynurenine, or 3-hydroxyanthranilic acid in the sample,
(2) step 2 of (i) calculating a ratio ((A)/(B)) of the amount (A) of anthranilic acid to the amount (B) of tryptophan, kynurenine, or 3-hydroxyanthranilic acid, or (ii) calculating a ratio ((B)/(A)) of the amount (B) of tryptophan, kynurenine, or 3-hydroxyanthranilic acid to the amount (A) of anthranilic acid,
   and
(3) step 3 of determining a psychiatric disorder based on a calculation result of the step 2.

### Psychiatric Disorder

The psychiatric disorder according to the present invention is a disorder in which a depressive state continues, and specific examples thereof include depressive disorder, bipolar disorder, schizophrenia, dementia, Alzheimer's disease, anxiety disorder, and epilepsy. Among these, the determining method of the present invention is useful for depressive disorder, bipolar disorder, schizophrenia, or dementia, and is particularly useful for depressive disorder.

In addition, depressive disorder is one of mood disorders and is a disorder characterized by symptoms such as depression, decreased mental activity, loss of appetite, and insomnia. In addition, the depressive disorder also includes major depressive disorder and dysthymic disorder.

### Sample

The sample according to the present invention may be a sample derived from a test animal, and examples thereof include living body-derived samples such as serum, plasma, whole blood, urine, saliva, cerebrospinal fluid, tissue fluid, sweat, tears, amniotic fluid, bone marrow fluid, pleural fluid, ascites fluid, synovial fluid, aqueous humor, and vitreous humor. A blood-derived sample such as serum, plasma, or whole blood, urine, or cerebrospinal fluid is preferable, a blood-derived sample such as serum, plasma, or whole blood is more preferable, and serum is particularly preferable.

Examples of the test animal include mammals such as humans, monkeys, mice, rats, dogs, cats, pigs, and rabbits. Humans, monkeys, mice, and rats are preferable, and humans are more preferable.

The method for obtaining (collecting) the sample according to the present invention from a test animal is not particularly limited. For example, a method for obtaining (collecting) the sample from the test animal based on a method known per se may be used, and separation, concentration, purification, and the like may be performed according to a method known per se, if necessary.

In addition, as the sample, a sample immediately after being collected from the test animal or the preserved sample may be used. As a method for preserving the sample, any method which is commonly used in this field may be used.

### Step 1

Step 1 according to the present invention is a step of measuring an amount of anthranilic acid in a sample and an amount of tryptophan, kynurenine, or 3-hydroxyanthranilic acid in a sample.

Tryptophan in the step 1 according to the present invention is a kind of amino acid, and is known that 90% thereof is metabolized through the Kynurenine pathway.

In addition, anthranilic acid, kynurenine, and 3-hydroxyanthranilic acid in the step 1 of the present invention are each a kind of amino acid, and are metabolites produced from tryptophan in the Kynurenine pathway that is the tryptophan metabolic pathway.

The structural formulas of anthranilic acid (the structural formula [1]), tryptophan (the structural formula [2]), kynurenine (the structural formula [3]), and 3-hydroxyanthranilic acid (the structural formula [4]) are shown below.

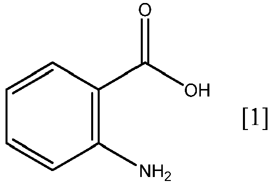

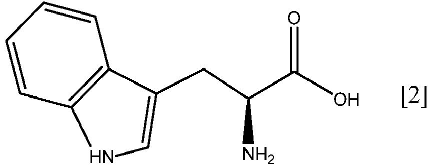

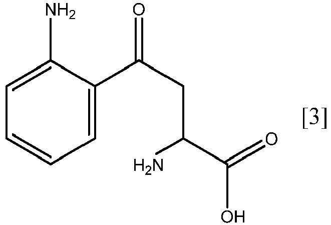

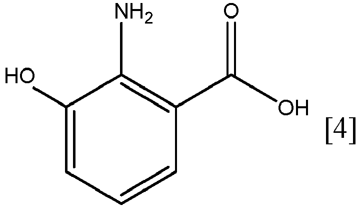

In the step 1 according to the present invention, as a method for measuring the amount of anthranilic acid, and the amount of tryptophan, kynurenine, or 3-hydroxyanthranil, any method may be used as long as the method is usually performed in this field. Specific examples of the method include a method using a mass spectrometer, an immunoassay, a method using NMR analysis, a method using an amino acid analyzer, and a FACS analysis. A method using a mass spectrometer or an immunoassay is preferable.

In a case of measuring the amount of anthranilic acid, and the amount of tryptophan, kynurenine, or 3-hydroxyanthranilic acid by the method using a mass spectrometer, the amounts thereof may be measured by injecting a mobile phase (such as an organic solvent-based mobile phase such as acetonitrile, methanol, tetrahydrofuran, isopropanol, or ethanol, and a water-based mobile phase such as water, phosphoric acid, formic acid, or acetic acid) and a sample into an apparatus provided with a column [such as an octadecylsilyl (ODS) column], a detector (such as a fluorescence detector, an electrochemical detector, or an ultraviolet visible spectroscopic detector), and separating and detecting anthranilic acid, and tryptophan, kynurenine, or 3-hydroxyanthranilic acid.

As a specific method of the method using a mass spectrometer, a method known per se may be used.

Specific examples of the method using a mass spectrometer include a method using high performance liquid chromatography (HPLC), a method using gas chromatography (GC), a method using a liquid chromatography mass spectrometer (LC/MS), a method using a capillary electrophoresis mass spectrometer (CE/MS), a method using a gas chromatography mass spectrometer (GC/MS), a method using an inductively coupled plasma mass spectrometer (ICP/MS), a method using a liquid chromatography tandem type mass spectrometer (LC/MS/MS), and a method using a gas chromatography tandem type mass spectrometer (GC/MS/MS), a method using high performance liquid chromatography (HPLC) or a method a liquid chromatography tandem type mass spectrometer (LC/MS/MS) is preferable, and a method using high performance liquid chromatography (HPLC) is more preferable.

In a case of measuring the amount of anthranilic acid, and the amount of tryptophan, kynurenine, or 3-hydroxyanthranilic acid by the immunoassay, the amounts thereof may be measured by using a substance having affinity with anthranilic acid, and a substance having affinity with tryptophan, a substance having affinity with kynurenine, or a substance having affinity with 3-hydroxyanthranilic acid to form a complex of anthranilic acid and the substance having affinity with anthranilic acid, and a complex of tryptophan and the substance having affinity with tryptophan, a complex of kynurenine and the substance having affinity with kynurenine, or a complex of 3-hydroxyanthranilic acid and the substance having affinity with 3-hydroxyanthranilic acid, respectively, and measuring each complex.

Regarding the specific method of the immunoassay, a method known per se may be used.

As the immunoassay, enzyme-linked immunosorbent assay (ELISA method), enzyme immunoassay (EIA method), radioimmunoassay (RIA method), fluorescent immunoassay (FIA method), chemiluminescent enzyme immunoassay (CLEIA method), electrochemiluminescence immunoassay (ECLEIA method), turbidimetric immunoassay, nephelometric Immunoassay, a latex agglutination method, immunochromatography, western blotting, luminescent oxygen channeling immunoassay (LOCI method), and liquid-phase binding assay-electrokinetic analyte transport assay (LBA-EATA method) may be used, and enzyme-linked immunosorbent assay (ELISA method), enzyme immunoassay (EIA method), radioimmunoassay (RIA method), fluorescent immunoassay (FIA method), chemiluminescent enzyme immunoassay(CLEIA method), electrochemiluminescence immunoassay (ECLEIA method), turbidimetric immunoassay, a latex agglutination method, or immunochromatography is preferable.

Specific examples of the substance having affinity with anthranilic acid, the substance having affinity with tryptophan, the substance having affinity with kynurenine and the substance having affinity with 3-hydroxyanthranilic acid include antibodies, lectins, polysaccharides, DNA, enzyme substrates, proteins, various receptors, and various ligands, and antibodies such as an anti-anthranilic acid antibody, an anti-tryptophan antibody, an anti-kynurenine antibody, and an anti-3-hydroxyanthranilic acid antibody are preferable.

The anti-anthranilic acid antibody, the anti-tryptophan antibody, the anti-kynurenine antibody, and the anti-3-hydroxyanthranilic acid antibody may be antibodies capable of specifically recognizing anthranilic acid, tryptophan, kynurenine, and 3-hydroxyanthranilic acid, respectively.

The antibodies may be polyclonal antibodies or monoclonal antibodies, and these may be used alone or in combination.

The antibodies may be antibody fragments such as Fab, F(ab'2), Fv, and sFv, or synthetic antibodies such as diabody, triabody, and tetrabody.

In addition, as the antibodies, commercially available antibodies may be used, or antibodies prepared according to method knowns per se may be used.

In addition, in a case of preparing the anti-anthranilic acid antibody, the anti-tryptophan antibody, the anti-kynurenine antibody, and the anti-3-hydroxyanthranilic acid antibody according to methods known per se, for example, the antibodies may be prepared according to the methods described in "Immunoassay" (edited by the Society for Biochemical Measurement, Kodansha, 2014) and JP2018-501202A.

Further, the anti-anthranilic acid antibody, the anti-tryptophan antibody, the anti-kynurenine antibody, and the anti-3-hydroxyanthranilic acid antibody may be labeled with labeling substances.

Specific examples of the labeling substances include enzymes such as horseradish peroxidase (HRP), calf intestinal alkaline phosphatase, and β-galactosidase, radioactive isotopes such as ^{99m}Tc, ¹³¹I, ¹²⁵I, ¹⁴C, ³H, ³²P, and ³⁵S, fluorescent substances such as fluorescein, fluorescein isothiocyanate (FITC), 4-methylumbelliferone, rhodamine, or derivatives thereof, luminescent substances such as luciferin, luminol, and ruthenium complex, substances having absorption in the ultraviolet region, such as phenol, naphthol, anthracene, or derivatives thereof, substances having properties as spin labeling agents typified by compounds with oxyl groups such as 4-amino-2,2,6,6-tetramethylpiperidine-1-oxyl, dyes such as HiLyte type dyes, Alexa type dyes, and CyDye type dyes, and nanoparticles such as colloidal gold and quantum dots.

In addition, as the method for binding the labeling substances to the anti-anthranilic acid antibody, anti-tryptophan antibody, anti-kynurenine antibody, and anti-3-hydroxyanthranilic acid antibody, any method known per se may be used.

The "amount" in the amount of anthranilic acid, and the amount of tryptophan, kynurenine, or 3-hydroxyanthranil may be an absolute value such as volume or mass, or a relative value such as concentration, ionic strength, light absorbance, fluorescence intensity, or turbidity. However, a relative value such as concentration, ionic strength, light absorbance, fluorescence intensity, or turbidity is preferable, and concentration is more preferable.

In the step 1 according to the present invention, as the reagent used for measuring the amount of anthranilic acid, and the amount of tryptophan, kynurenine, or 3-hydroxyanthranilic acid, any reagent commonly used in this field may be used and the concentration and the pH thereof may be in a range commonly used in this field.

In addition, as the condition and operation method of the apparatus, any condition and operation method may be used as long as the condition and the operation method are commonly used in this field.

In the step 1 according to the present invention, it is preferable to measure the amount of anthranilic acid and the amount of kynurenine or 3-hydroxyanthranilic acid, and it is more preferable to measure the amount of anthranilic acid and the amount of 3-hydroxyanthranilic acid.

### Step 2

Step 2 according to the present invention is a step of (i) calculating a ratio of the amount of anthranilic acid to the amount of tryptophan, kynurenine, or 3-hydroxyanthranilic acid, or (ii) calculating a ratio of the amount of tryptophan, kynurenine, or 3-hydroxyanthranilic acid to the amount of anthranilic acid.

Specifically, the step 2 according to the present invention is performed by (i) dividing the amount of anthranilic acid obtained in the step 1 according to the present invention by the amount of tryptophan, kynurenine, or 3-hydroxyanthranilic acid obtained in the step 1 according to the present invention, or (ii) dividing the amount of tryptophan, kynurenine, or 3-hydroxyanthranilic acid obtained in the step 1 according to the present invention by the amount of anthranilic acid obtained in the step 1 according to the present invention.

In the step 2 according to the present invention, it is preferable to calculate a ratio of the amount of anthranilic acid to the amount of tryptophan, kynurenine, or 3-hydroxyanthranilic acid, it is more preferable to calculate a ratio of the amount of anthranilic acid to the amount of kynurenine or 3-hydroxyanthranilic acid, and it is particularly preferable to calculate a ratio of the amount of anthranilic acid to the amount of 3-hydroxyanthranilic acid.

### Step 3

Step 3 according to the present invention is a step of determining a psychiatric disorder based on the calculation result of the step 2 according to the present invention.

Specifically, the step 3 according to the present invention is performed using values obtained by the step 1 and the step 2 according to the present invention using a sample derived from a test animal (hereinafter, sometimes abbreviated as a value derived from a test animal) and a preset reference value (cutoff value or the like).

That is, (i) in a case where the value derived from a test animal is equal to or greater than a preset reference value (cutoff value or the like), it can be determined that "there is a risk of psychiatric disorder in the test animal or a high risk of psychiatric disorder in the test animal". On the other hand, (ii) in a case where the value derived from a test animal is less than a preset reference value (cutoff value or the like), it can be determined that "there is no risk of psychiatric disorder in the test animal or there is a low risk of psychiatric disorder".

In addition, as another aspect, (i) in a case where the value derived from a test animal is equal to or less than a preset reference value (cutoff value or the like), it can be determined that "there is a risk of psychiatric disorder in the test animal or there is a high risk of psychiatric disorder in the test animal". On the other hand, (ii) in a case where the value derived from a test animal is greater than a preset reference value (cutoff value or the like), it can be determined that "there is no risk of psychiatric disorder in the test animal or there is a low risk of psychiatric disorder in the test animal".

The above reference value (cutoff value or the like) can be determined based on statistical analysis such as receiver operating characteristic (ROC) curve analysis by using the values obtained in the step 1 and the step 2 according to the present invention using a sample derived from an animal suffering from a psychiatric disorder and the values obtained in the step 1 and the step 2 using a sample derived from a healthy animal. In addition, the sensitivity or/and specificity of the reference value (cutoff value or the like) is, for example, 60% or more, preferably 70% or more, more preferably 80% or more, even more preferably 90% or more, and particularly preferably 95% or more.

### Specific Example of Determining Method of the Present Invention

A specific example of the determining method of the present invention will be described below.

### (1) Step 1 According to the Present Invention

After 10 to 200 µL of a sample (for example, serum) is extracted from a test animal (for example, human) and 25 to 800 µL of a protein denaturing agent (for example, 5% to 10% of perchloric acid) is added, centrifugation is performed at 10000 to 15000 rpm and 4°C for 5 to 15 minutes. Then, 40 to 60 µL of a supernatant is injected into a mass spectrometer provided with a reversed phase column such as an ODS column (for example, high performance liquid chromatography apparatus), and a basic solution (for example, 5 to 15 mM of sodium acetate) and an organic solvent (for example, acetonitrile) are used as a mobile phase to flow the solution at a flow rate of 0.1 to 1 mL/min to separate the components in the sample. Next, the amount of anthranilic acid and the amount of tryptophan, kynurenine, or 3-hydroxyanthranilic acid in the supernatant are measured using a detector (for example, an ultraviolet visible spectroscopic detector, a fluorescence detector, or an electrochemical detector).

### (2) Step 2 According to the Present Invention

A ratio is calculated by dividing the amount of anthranilic acid derived from the test animal measured in (1) by the amount of tryptophan, kynurenine, or 3-hydroxyanthranilic acid.

### (3) Step 3 According to the Present Invention

In a case where the value derived from the test animal calculated by (2) above is compared with a preset reference value (cutoff value or the like) and the value derived from the test animal is equal to or greater than the preset reference value (cutoff value or the like), it is determined that "there is a risk of psychiatric disorder in the test animal or there is a high risk of psychiatric disorder in the test animal". On the other hand, in a case where the value derived from the test animal calculated in (2) above is compared with a preset reference value (cutoff value or the like) and the value derived from the test animal is less than the preset reference value (cutoff value or the like), it is determined that "there is no risk of psychiatric disorder in the test animal or there is a low risk of psychiatric disorder in the test animal".

In addition, as another aspect, in a case where the value derived from the test animal calculated in (2) above is compared with a preset reference value (cutoff value or the like) and the value derived from the test animal is equal to or less than the preset reference value (cutoff value or the like), it is determined that "there is a risk of psychiatric disorder in the test animal or there is a high risk of psychiatric disorder in the test animal".

On the other hand, in a case where the value derived from the test animal calculated in (2) above is compared with the preset reference value (cutoff value or the like), and the value derived from the test animal is greater than the preset reference value (cutoff value or the like), it is determined that "there is no risk of psychiatric disorder in the test animal or there is a low risk of psychiatric disorder in the test animal".

### Method for Obtaining Data for Determining Psychiatric Disorder of the Present Invention

The method for obtaining data for determining a psychiatric disorder of the present invention (hereinafter, sometimes abbreviated as the method for obtaining data of the present invention) comprises (1) step 1 of measuring an amount (A) of anthranilic acid in a sample and an amount (B) of tryptophan, kynurenine, or 3-hydroxyanthranilic acid in the sample, and
(2) step 2 of (i) calculating a ratio ((A)/(B)) of the amount (A) of anthranilic acid to the amount (B) of tryptophan, kynurenine, or 3-hydroxyanthranilic acid, or (ii) calculating a ratio ((B)/(A)) of the amount (B) of tryptophan, kynurenine, or 3-hydroxyanthranilic acid to the amount (A) of anthranilic acid.

Examples of the data in the method for obtaining data of the present invention include (i) the value calculated in the step 2 according to the method for obtaining data of the present invention, (ii) a value obtained by further subjecting the value to multivariate analysis such as multiple logistic regression analysis, discriminant analysis, Poisson regression analysis, multiple regression analysis, Cox proportional hazards model, or path analysis, (iii) data showing the magnitude relationship between the value calculated in the step 2 according to the method for obtaining data of the present invention and the reference value (cutoff value or the like) (comparison result), and (iv) data indicating that there is a risk of psychiatric disorder in the test animal or there is a high risk of psychiatric disorder in the test animal. (i) or (iii) is preferable, and (i) is more preferable.

In addition, the terms [sample], [psychiatric disorder], [step 1], and [step 2] in the method for obtaining data of the present invention are as described in the <Determining Method of the Present Invention>, and preferable examples, specific examples, and the like are also the same.

### Marker for Determining Psychiatric Disorder of the Present Invention

A marker for determining psychiatric disorder of the present invention (hereinafter, sometimes abbreviated as a marker of the present invention) comprises anthranilic acid and tryptophan, kynurenine, or 3-hydroxyanthranilic acid.

In addition, the term [psychiatric disorder] in the marker of the present invention is as described in the <Determining Method of the Present Invention>, and preferable examples, specific examples and the like are also the same.

The marker of the present invention preferably contains anthranilic acid and kynurenine or 3-hydroxyanthranilic acid, and more preferably contains anthranilic acid and 3-hydroxyanthranilic acid.

### Apparatus for Determining Psychiatric Disorder According to the Present Invention

An apparatus for determining psychiatric disorder according to the present invention (hereinafter, sometimes abbreviated as a determining apparatus according to the present invention) is provided with at least (1) measurement unit, and (2) processing unit. Further, the determining apparatus may be provided with (3) determining unit, (4) output unit, and (5) input unit.

(1) Measurement unit in the determining apparatus according to the present invention is configured to measure an amount (A) of anthranilic acid in a sample, and an amount (B) of tryptophan, kynurenine, or 3-hydroxyanthranilic acid in the sample. Specific examples thereof include measurement apparatuses such as a mass spectrometer used in the above measuring method, a device used in an immunoassay, an NMR analyzer, an amino acid analyzer, and a device used in FACS analysis.

In the determining apparatus according to the present invention, (2) processing unit is configured to (i) calculate a ratio ((A)/(B)) of the amount (A) of anthranilic acid to the amount (B) of tryptophan, kynurenine, or 3-hydroxyanthranilic acid measured by (1) measurement unit or (ii) calculate a ratio ((B)/(A)) of the amount (B) of tryptophan, kynurenine, or 3-hydroxyanthranilic acid to the amount (A) of anthranilic acid. In addition, if necessary, the processing unit may be configured to calculate the amount of anthranilic acid, and the amount of tryptophan, kynurenine, or 3-hydroxyanthranilic acid based on the measurement values measured by (1) measurement unit and calculate the ratio ((A)/(B) or (B)/(A)) based on the calculation result.
(3) Determining unit in the determining apparatus according to the present invention is configured to determine a psychiatric disorder based on the calculation result obtained by (2) processing unit.
(4) Output unit in the determining apparatus according to the present invention is configured to output the calculation result obtained by (2) processing unit and/or the determinatin result obtained by (3) determining unit.
(5) Input unit in the determining apparatus according to the present invention is configured to send a signal to (1) measurement unit or/and (2) processing unit to activate (1) measurement unit or/and (2) processing unit by receiving the operation of an operator.

In addition, the measurement, calculation, and determination using (1) measurement unit, (2) processing unit, and (3) determining unit of the determining apparatus according to the present invention may be performed according to the <Determining Method of the Present Invention>, and preferable examples, specific examples, and the like are also made according to the determining method.

According to the determining apparatus of the present invention, the determining method of the present invention and/or the method for obtaining data of the present invention can be easily and accurately performed in a short period of time.

### Method for Performing Measurement and Calculation for Determining Psychiatric Disorder According to the Present Invention

A method for performing measurement and calculation for determining a psychiatric disorder according to the present invention comprises, in order to determine a psychiatric disorder, (1) a measurement step of measuring an amount (A) of anthranilic acid in a sample, and an amount (B) of tryptophan, kynurenine, or 3-hydroxyanthranilic acid in the sample, and
(2) a calculation step of (i) calculating a ratio ((A)/(B)) of the amount (A) of anthranilic acid to the amount (B) of tryptophan, kynurenine, or 3-hydroxyanthranilic acid, or (ii) calculating a ratio ((B)/(A)) of the amount (B) of tryptophan, kynurenine, or 3-hydroxyanthranilic acid to the amount (A) of anthranilic acid. In addition, in the calculation step, if necessary, the amount of anthranilic acid, and the amount of tryptophan, kynurenine, or 3-hydroxyanthranilic acid in the sample may be calculated based on the measurement values obtained as results of the measurement in (1) to calculate the ratio ((A)/(B) or (B)/(A)) based on the calculation results.

In addition, the measurement step and the calculation step in the method of performing measurement and calculation of the present invention are as described in the <Determining Method of the Present Invention>, and preferable examples, specific examples, and the like are also the same.

### Method for Assisting Determination of Psychiatric Disorder According to the Present Invention

A method for assisting the determination of a psychiatric disorder according to the present invention (hereinafter, sometimes abbreviated as an assisting method according to the present invention) includes (1) step 1 of measuring an amount (A) of anthranilic acid in a sample, and an amount (B) of tryptophan, kynurenine, or 3-hydroxyanthranilic acid in the sample,
(2) step 2 of (i) calculating a ratio ((A)/(B)) of the amount (A) of anthranilic acid to the amount (B) of tryptophan, kynurenine, or 3-hydroxyanthranilic acid, or (ii) calculating a ratio ((B)/(A)) of the amount (B) of tryptophan, kynurenine, or 3-hydroxyanthranilic acid to the amount (A) of anthranilic acid,
   and
(3) step 3 of assisting determination of a psychiatric disorder based on the calculation result of the step 2. The assisting method according to the present invention can be used as a method for assisting the diagnosis of a psychiatric disorder by a doctor or the like.

In addition, the terms [sample], [psychiatric disorder], [step 1], [step 2], and [step 3] in the method for obtaining data of the present invention are as described in the <Determining Method of the Present Invention>, and preferable examples, specific examples, and the like are also the same.

In addition, in the step 2, if necessary, the amount of anthranilic acid, and the amount of tryptophan, kynurenine, or 3-hydroxyanthranilic acid in the sample may be calculated based on the measurement values obtained as results of the measurement in (1), and the ratio ((A)/(B) or (B)/(A)) may be calculated based on the calculation results.

### Method for Determining and Treating Psychiatric Disorder According to the Present Invention

A method for determining and treating a psychiatric disorder according to the present invention (hereinafter, sometimes abbreviated as a treatment method according to the present invention) includes (1) step 1 of measuring an amount (A) of anthranilic acid in a sample, and an amount (B) of tryptophan, kynurenine, or 3-hydroxyanthranilic acid in the sample,
(2) step 2 of (i) calculating a ratio ((A)/(B)) of the amount (A) of anthranilic acid to the amount (B) of tryptophan, kynurenine, or 3-hydroxyanthranilic acid, or (ii) calculating a ratio ((B)/(A)) of the amount (B) of tryptophan, kynurenine, or 3-hydroxyanthranilic acid to the amount (A) of anthranilic acid, (3) step 3 of determining a psychiatric disorder based on a calculation result of the step 2,
   and
(4) step 4 of appropriately treating a patient who is determined to have a risk of psychiatric disorder or a high risk of psychiatric disorder based on the determination result of the step 3.

In addition, the terms [sample], [psychiatric disorder], [step 1], [step 2], and [step 3] in the treatment method for the present invention are as described in the <Determining Method of the Present Invention>, and preferable examples, specific examples, and the like are also the same. In addition, in the step 2, if necessary, the amount of anthranilic acid, and the amount of tryptophan, kynurenine, or 3-hydroxyanthranilic acid in the sample may be calculated based on the measurement values obtained as results of the measurement in (1), and the ratio ((A)/(B) or (B)/(A)) may be calculated based on the calculation results. As the appropriate treatment in the step 4 of the treatment method according to the present invention, psychotherapy including an interview with a medical staff such as a doctor, pharmacotherapy that is performed by administering an antidepressant such as amoxapine (trade name), maprotiline (trade name), fluvoxamine (trade name), paroxetine (trade name), milnacipran (trade name), or mirtazapine (trade name), modified electroconvulsive therapy, family therapy, and rehabilitation.

### Reagent for Determining Psychiatric Disorder According to the Present Invention

A reagent for determining a psychiatric disorder according to the present invention (hereinafter, sometimes abbreviated as a reagent according to the present invention) includes a reagent for detecting the marker of the present invention as a constituent element.

Examples of the reagent for detecting the marker of the present invention include those containing a substance having affinity with the marker of the present invention, and specifically, for example, antibodies, lectins, polysaccharides, nucleic acids, enzymes, proteins, and the like may be used. Antibodies such as an anti-anthranilic acid antibody, an anti-tryptophan antibody, an anti-kynurenine antibody, and an anti-3-hydroxyanthranilic acid antibody are preferable.

In addition, the antibodies are as described in the <Determining Method of the Present Invention>, and preferable examples and specific examples are also the same.

The reagent according to the present invention may includes reagents commonly used in this field, and examples thereof include buffers, detergents, reaction promoters, saccharides, proteins, salts, stabilizers such as surfactants, preservatives, liquids for diluting samples, immobilized solid phases such as antibody-immobilized solid phases and antigen-immobilized solid phases, secondary antibodies labeled with labeling substances or antibody fragments thereof, and reagents for detecting labeling substances. A reagent that does not impair stability of a coexisting reagent or the like may be used. In addition, the concentration and pH thereof may be in the ranges commonly used in this field.

As the immobilized solid phases such as antibody-immobilized solid phases and antigen-immobilized solid phases, any immobilized solid phases may be used as long as the immobilized solid phases are those obtained by immobilizing anthranilic acid, tryptophan, kynurenine, or 3-hydroxyanthranilic acid, or antibodies such as an anti-anthranilic acid antibody, an anti-tryptophan antibody, an anti-kynurenine antibody, and an anti-3-hydroxyanthranilic acid antibody, or antibody fragments thereof to materials such as magnetic particles such as magnetic silica particles, polystyrene, polycarbonate, polyvinyltoluene, polypropylene, polyethylene, polyvinyl chloride, nylon, polymethacrylate, gelatin, agarose, cellulose, polyethylene terephthalate, glass, and ceramics.

As the materials for the immobilized solid phases such as antibody-immobilized solid phases, and antigen-immobilized solid phases, materials produced by method knowns per se or commercially available materials may be used. For example, in a case where the magnetic particles such as magnetic silica particles are produced by a method known per se, the magnetic particles can be produced by the method described in WO2012/173002.

The secondary antibodies labeled with labeling substances or the antibody fragments thereof are antibodies or antibody fragments thereof to be bonded to an anti-anthranilic acid antibody, an anti-tryptophan antibody, an anti-kynurenine antibody, and an anti-3-hydroxyanthranilic acid antibody, respectively.

In addition, the labeling substances and the method for binding the labeling substances in the secondary antibodies labeled with labeling substances or the antibody fragments thereof are as described in the <Determining Method of the Present Invention>, and preferable examples, specific examples, and the like are also the same.

The reagent for detecting a labeling substance is a reagent that detects, in a case where an antibody such as an anti-anthranilic acid antibody, an anti-tryptophan antibody, an anti-kynurenine antibody, or an anti-3-hydroxyanthranilic acid antibody is labeled with a labeling substance, the label in the labeled antibody such as an anti-anthranilic acid antibody, an anti-tryptophan antibody, an anti-kynurenine antibody, or an anti-3-hydroxyanthranilic acid antibody or an antibody fragment thereof, and/or the label in the labeled secondary antibody or an antibody fragment thereof, and examples thereof include substrates for measuring absorbance such as tetramethylbenzidine and orthophenylenediamine, fluorescent substrates such as hydroxyphenylpropionic acid and hydroxyphenylacetic acid, luminescent substances such as luminol, and further includes reagents for measuring absorbance such as 4-nitrophenyl phosphate and fluorescent substrates such as 4-methylumbelliferyl phosphate.

Further, the reagent according to the present invention may include an instruction for performing the determining method and/or the method for obtaining data of the present invention. The "instruction" means an instruction manual, attached document, or pamphlet (leaflet) for the reagent according to the present invention in which the features, principles, operating procedure, determination procedure, and the like of the determining method or/and the method for obtaining data of the present invention are substantially disclosed in sentences and/or using diagrams.

As described above, according to the reagent of the present invention, the determining method of the present invention, and/or the method for obtaining data of the present invention can be easily and accurately performed in a short period of time.

Hereinafter, the present invention will be described more specifically based on examples, but the present invention is not limited to the examples.

### Examples

### Example 1. Analysis of Tryptophan and Its Metabolites in Depression Group and Healthy Group

### (1) Sample

The serums of examinees corresponding to the following "depression group" or "healthy group" were used as samples from 2080 examinees excluding 5 examinees with defective inspection results from 2085 human dock examinees.

### • Depression Group

Among the 2080 examinees, 42 examinees (28 males and 14 females) were classified into a depression group according to the following classification method.

That is, among 60 examinees tested positive in depression in all three tests [center for epidemiologic studies depression scale (CES-D), general health questionnaire (GHQ28), and brief structured interview for depression (BSID)] aimed at evaluating depression symptoms, 6 examinees who were taking an antidepressant were excluded, further, the age with the healthy group described later was adjusted, and 42 examinees (28 males and 14 females) of the 2080 examinees were classified into the depression group.

The results of CES-D, GHQ28 and BSID tests are shown in Table 1. In addition, in the table, "+" indicates positive and "-" indicates negative.

**[Table 1]**

| CES-D | GHQ28 | BSID | Number of people |
|---|---|---|---|
| + | + | + | 60 |
| + | + | - | 198 |
| - | + | + | 3 |
| + | - | + | 3 |
| + | - | - | 91 |
| - | + | - | 206 |
| - | - | + | 0 |
| - | - | - | 1429 |

The results in Table 1 show the results of 1990 examinees who did not have any defects in the test answers among the 2085 clinical survey examinees. In the CES-D test, a score of 16 or higher was considered positive in depression according to the guidelines thereof, and in the GHQ28 test, a score of 8 or higher was considered positive in depression according to the "The Psychological Assessment Handbook (Nishimura Shoten Co. Ltd; 2001)".

In addition, in the BSID test, in a case where at least one of Q1 or Q2 in Table 2 below was "Yes", the answers of Q3 and Q4 in Table 2 below were promoted, and in a case where at least one of Q3 or Q4 was "Yes", this case was considered positive in depression.

**[Table 2]**

| | |
|---|---|
| **Q1** | Do you feel depressed or feel down all day long almost every day? Has the feeling continued for two weeks or longer? |
| **Q2** | Do you lose interest in most things or are you no longer enjoying what you normally enjoy? Has the feeling continued for two weeks or longer? |
| **Q3** | In a case where you have felt down or not have been interested in most things for two weeks or longer, do you feel worthless of yourself or guilty almost every day? |
| **Q4** | Do you feel difficulty in focusing or making decisions almost every day? |

### • Healthy Group

Among the 2080 examinees, 42 examinees (25 males and 17 females) were classified into a healthy group according to the following classification method.

That is, among 61 examinees who satisfied the conditions in Table 3 below, the age with the depression group was adjusted, and 42 examinees (25 males and 17 females) among the 2080 examinees were classified into a healthy group.

**[Table 3]**

| Item | Numerical value, Result | Item | Numerical value, Result |
|---|---|---|---|
| Regular medication | None | LDL cholesterol | < 140 mmHg/dL |
| Smoking | Non-smoking | Neutral fat | < 150 mg/dL |
| Alcohol consumption | Less than 20 g/day | HDL cholesterol | ≧ 40 mg/dL |
| BMI | <25 | CES-D score | < 16 points |
| Systolic blood pressure | < 140 mmHg | GHQ28 score | < 8 points |
| Diastolic blood pressure | < 90 mmHg | BSID result | Negative in depression |
| Fasting blood sugar | < 110 mg/dL | - | - |

### (2) Measurement of Tryptophan and Its Metabolites in Serum Derived From Depression group And Healthy Group

The concentrations of tryptophan and its metabolites (kynurenine, 3-hydroxyanthranilic acid, kynurenic acid, anthranilic acid, and 3-hydroxykynurenine) in the serum derived from the depression group and the healthy group were measured by the following method.

### Measurement of Tryptophan, Kynurenine, 3-Hydroxyanthranilic Acid, Kynurenic Acid, and Anthranilic Acid

Deproteinization was performed by adding 25 µL of 10% perchloric acid to 100 µL of serum derived from the depression group and the healthy group. Next, after centrifugation was performed at 14000 rpm and 4°C for 10 minutes, 50 µL of a supernatant was injected into a high performance liquid chromatography [LC-20AD (manufactured by Shimadzu Corporation)], tryptophan and kynurenine in the supernatant were measured by an ultraviolet visible spectroscopic detector [SPD-20A (manufactured by Shimadzu Corporation), and 3-hydroxyanthranilic acid, kynurenic acid, and anthranilic acid in the supernatant were measured with a fluorescence detector [RF-10AXL (manufactured by Shimadzu Corporation)].

The conditions of the high performance liquid chromatography and the detector are as follows.
- High performance liquid chromatography [LC-20AD (manufactured by Shimadzu Corporation)]
   Mobile phase: Aqueous solution containing 10 mM sodium acetate and 0.5% to 1.25% acetonitrile (pH 4.5)
   Flow rate: 0.9 mL/min
   Column: TSKgel ODS-100V, 3 µm, 4.6 mm (ID) × 15 cm (L) [manufactured by Tosoh Corporation]
   Column temperature: 40°C
- Detector
   Ultraviolet visible spectroscopic detector [SPD-20A (manufactured by Shimadzu Corporation)]:
      Tryptophan → UV wavelength: 280 nm, kynurenine → UV wavelength: 365 nm
   Fluorescence detector [RF-10AXL (manufactured by Shimadzu Corporation)]: 3-hydroxyanthranilic acid and anthranilic acid → excitation wavelength: 320 nm, fluorescence wavelength: 420 nm, kynurenic acid → excitation wavelength: 334 nm, fluorescence wavelength: 380 nm

### Measurement of 3-Hydroxykynurenine

Deproteinization was performed by adding 400 µL of 10% perchloric acid to 100 µL of serum derived from the depression group and the healthy group. Next, after centrifugation was performed at 14000 rpm and 4°C for 10 minutes, 50 µL of a supernatant was injected into a high performance liquid chromatography (manufactured by Eicom Corporation), and 3-hydroxykynurenine in the supernatant was measured by an electrochemical detector [Eicom ECD-300 (manufactured by Eicom Corporation)].

The conditions of the high performance liquid chromatography and the electrochemical detector are as follows.
High performance liquid chromatography (manufactured by Eicom Corporation)
Mobile phase: Aqueous solution containing 0.27 mM EDTA, 8.9 mM M1-heptane sodium sulfonate, 0.59% phosphoric acid, 0.9% triethylamine, and 1% acetonitrile
Flow rate: 0.5 mL/min
Column: EICOMPAK SC-50DS 3.0 mm (ID) × 150 mm (L) [manufactured by Eicom Corporation]
Column temperature: 25°C
   - Electrochemical detector [Eicom ECD-300 (manufactured by Eicom Corporation)] Applied voltage: + 500 mV

### (3) Results

Table 4 shows the measurement results (mean values) of the healthy group and the depression group obtained in (2) above, P values obtained by a significant difference determination, and the presence or absence of the significant difference. Further, Table 5 shows the results of calculating respective ratios of the amount of anthranilic acid to the amount of tryptophan, the amount of anthranilic acid to the amount of kynurenine, the amount of anthranilic acid to the amount of 3-hydroxyanthranilic acid, the amount of anthranilic acid to the amount of kynurenic acid, the amount of anthranilic acid to the amount of 3-hydroxykynurenine, the amount of kynurenine to the amount of tryptophan, the amount of kynurenic acid to the amount of kynurenine, the amount of 3-hydroxykynurenine to the amount of kynurenine, and the amount of 3-hydroxyanthranilic acid to the amount of 3-hydroxykynurenine, using the measurement values of the healthy group and the depression group obtained in (2) above.

It is noted that, all the mean values in the table are expressed as mean ± standard deviation (mean ± SD). In addition, the t-test was used for the determination of the significant difference, and those having a P value of less than 0.05 (P < 0.05) were determined to have the significant difference.

**[Table 4]**

| | Males and Females | | | |
|---|---|---|---|---|
| | Healy group (n=42) | Depression group (n=42) | P Value | Significant difference |
| Anthranilic acid (nM) | 9.495±3.567 | 14.669±6.988 | 0.000052 | **** Presence (P<0.0001) |
| Tryptophan (µM) | 51.994±7.205 | 47.634±7.301 | 0.0072 | ** Presence (P<0.01) |
| Kynurenine (µM) | 1.527±0.249 | 1.443±0.321 | 0.1834 | Absence |
| 3-Hydroxyanthranilic acid (nM) | 14.271±5.359 | 13.305±5.920 | 0.4355 | Absence |
| Kynurenic acid (nM) | 23.625±5.806 | 27.127±10.087 | 0.0546 | Absence |
| 3-Hydroxykynurenine (nM) | 17.466±6.919 | 15.409±8.419 | 0.4164 | Absence |

**[Table 5]**

| | | Males and Females | | | |
|---|---|---|---|---|---|
| | | Healthy group (n=42) | Depression group(n=42) | P Value | Significant difference |
| A | Anthranilic acid/ tryptophan | 0.00019±0.000084 | 0.000313±0.000157 | 0.000021 | **** Presence (P<0.0001) |
| B | Anthranilic acid/ kynurenine | 0.00627±0.0023 | 0.0104±0.00517 | 0.0000072 | **** Presence (P<0.0001) |
| C | Anthranilic acid/ 3-Hydroxyanthranilic acid | 0.719±0.275 | 1.225±0.590 | 0.0000028 | **** Presence (P<0.0001) |
| D | Anthranilic acid/ kynurenic acid | 0.418±0.165 | 0.576±0.244 | 0.00082 | *** Presence (P<0.001) |
| E | Anthranilic acid/ 3-Hydroxykynurenine | 0.648±0.227 | 1.230±0.819 | 0.010 | * Presence (P<0.05) |
| F | Kynurenine/ tryptophan | 0.02984±0.0061 | 0.0303±0.00453 | 0.7268 | Absence |
| G | Kynurenic acid/ kynurenine | 0.0156±0.0038 | 0.0187±0.00573 | 0.0045 | ** Presence (P<0.01) |
| H | 3-Hydroxykynurenine/ kynurenine | 0.0119±0.0041 | 0.0117±0.00833 | 0.879 | Absence |
| I | 3-Hydroxyanthranilic acid/ 3-Hydroxykynurenine | 1.0155±0.442 | 1.0404±0.548 | 0.879 | Absence |

From Table 4, it was found that the amount of anthranilic acid, the amount of tryptophan, and the amount of kynurenic acid in the serum were increased in the depression group compared with the healthy group. Among these, it was found that the amount of anthranilic acid was significantly increased (P = 0.000052).

On the other hand, it was found that the amount of kynurenine, the amount of 3-hydroxyanthranilic acid, and the amount of 3-hydroxykynurenine in the serum were decreased in the depression group compared with the healthy group, but no significant difference was observed.

From Table 5, it was found that the values of the ratios of the amount of anthranilic acid to the amount of tryptophan, the amount of anthranilic acid to the amount of kynurenine, the amount of anthranilic acid to the amount of 3-hydroxyanthranilic acid, the amount of anthranilic acid to the amount of kynurenic acid, the amount of anthranilic acid to the amount of 3-hydroxykynurenine, the amount of kynurenine to the amount of tryptophan, the amount of kynurenic acid to the amount of kynurenine, and the amount of 3-hydroxyanthranilic acid to the amount of 3-hydroxykynurenine [(A) to (G) and (I) in Table 5] were increased in the depression group compared to the healthy group. Among these, it was found that the values of the ratios of the amount of anthranilic acid to the amount of tryptophan, the amount of anthranilic acid to the amount of kynurenine, and the amount of anthranilic acid to the amount of 3-hydroxyanthranilic acid [(A) to (C) in Table 5] were particularly significantly increased (P = 0.000021, P = 0.0000072, and P = 0.0000028, respectively).

On the other hand, it was found that the value of the ratio of the amount of 3-hydroxykynurenine to the amount of kynurenine [(H) in Table 5] was decreased in the depression group compared with the healthy group, but no significant difference was observed.

From Tables 4 and 5, it is found that compared with the healthy group, in the depression group, an increase in the values of the ratios of the amount of anthranilic acid to the amount of tryptophan, the amount of anthranilic acid to the amount of the kynurenine, and the amount of anthranilic acid to the amount of 3-hydroxyanthranilic acid was more significant than an increase in the value of the amount of anthranilic acid alone.

That is, the P value showing a significant difference in the increase in the value of the amount of anthranilic acid alone in the depression group is 0.000052. On the other hand, the P values showing the significance of the increase in the values of the ratios of the amount of anthranilic acid to the amount of tryptophan, the amount of anthranilic acid to the amount of kynurenine, and the amount of anthranilic acid to the amount of 3-hydroxyanthranilic acid in the depression group are 0.000021, 0.0000072, and 0.0000028, respectively. [Table 5 (A) to (C)]. Particularly, the P value showing the significance of the increase in the values of the ratios of the amount of anthranilic acid to the amount of kynurenine, and the amount of anthranilic acid to the amount of 3-hydroxyanthranilic acid is a value of about 1/10 of the P value showing the significant difference of the increase in the value of the amount of anthranilic acid alone.

Accordingly, it was found that the values of the ratios of the amount of anthranilic acid to the amount of tryptophan, the amount of anthranilic acid to the amount of kynurenine, and the amount of anthranilic acid to the amount of 3-hydroxyanthranilic acid were more useful in distinguishing between the depression group and the healthy group than the value of the amount of anthranilic acid alone.

In addition, from Table 5, the P values showing the significance of the increase in the values of the ratios of the amount of anthranilic acid to the amount of kynurenic acid and the amount of anthranilic acid to the amount of 3-hydroxykynurenine are 0.00082 and 0.010, respectively [(D) and (E) in Table 5]. In addition, the P-values showing the significance of the increase in the values of the ratios of the amount of kynurenine to the amount of tryptophan, the amount of kynurenic acid to the amount of kynurenine, and the amount of 3-hydroxyanthranilic acid to the amount of 3-hydroxykynurenine are 0.7268, 0.0045, and 0.879, respectively. [(F) to (H) in Table 5].

Accordingly, a specific combination of anthranilic acid and substances related to tryptophan, that is, a combination of anthranilic acid and tryptophan, anthranilic acid and kynurenine, or anthranilic acid and 3-hydroxyanthranilic acid is useful in distinguishing between the depression group and the healthy group.

In other words, it is possible to determine (diagnose and inspect) depressive disorder with high reliability (accuracy and precision) by calculating the ratio of the amount of anthranilic acid to the amount of tryptophan, the amount of anthranilic acid to the amount of kynurenine, or the amount of anthranilic acid to the amount of 3-hydroxyanthranilic acid.

In addition, the depression group in the example is a group before a doctor makes a diagnosis of depressive disorder. Therefore, according to the determining method of the present invention, it is possible to determine whether or not the examinee has depressive disorder at an early stage.

Further, the monitoring of a change in the value of the ratio of the amount of anthranilic acid and the amount of tryptophan, kynurenine, or 3-hydroxyanthranilic acid calculated by the determining method of the present invention can also be used to determine the therapeutic effect of depressive disorder.

### Industrial Applicability

According to the method for determining a psychiatric disorder and the marker for determining a psychiatric disorder of the present invention, it is possible to determine (diagnose and inspect) a psychiatric disorder such as depressive disorder, bipolar disorder, schizophrenia, or dementia with high reliability (accuracy and precision).

## Claims

1. A method for determining a psychiatric disorder comprising the following steps (1) to (3):
(1) step 1 of measuring an amount (A) of anthranilic acid in a sample, and an amount (B) of tryptophan, kynurenine, or 3-hydroxyanthranilic acid in the sample;
(2) step 2 of (i) calculating a ratio ((A)/(B)) of the amount (A) of anthranilic acid to the amount (B) of tryptophan, kynurenine, or 3-hydroxyanthranilic acid, or (ii) calculating a ratio ((B)/(A)) of the amount (B) of tryptophan, kynurenine, or 3-hydroxyanthranilic acid to the amount (A) of anthranilic acid; and
(3) step 3 of determining a psychiatric disorder based on a calculation result of the step 2.

2. The determining method according to claim 1,
wherein the step 2 is a step of (i) calculating a ratio ((A)/(B)) of the amount (A) of anthranilic acid to the amount (B) of tryptophan, kynurenine, or 3-hydroxyanthranilic acid.

3. The determining method according to claim 1,
wherein the step 2 is a step of calculating a ratio of the amount of anthranilic acid to the amount of kynurenine or 3-hydroxyanthranilic acid.

4. The determining method according to claim 1,
wherein the psychiatric disorder is depressive disorder, bipolar disorder, schizophrenia, or dementia.

5. The determining method according to claim 1,
wherein the psychiatric disorder is depressive disorder.

6. The determining method according to claim 1,
wherein the sample is serum, plasma, whole blood, urine, or cerebrospinal fluid.

7. A method for obtaining data for determining a psychiatric disorder, the method comprising the following steps (1) and (2):
(1) step 1 of measuring an amount (A) of anthranilic acid in a sample, and an amount (B) of tryptophan, kynurenine, or 3-hydroxyanthranilic acid in the sample; and
(2) step 2 of (i) calculating a ratio ((A)/(B)) of the amount (A) of anthranilic acid to the amount (B) of tryptophan, kynurenine, or 3-hydroxyanthranilic acid, or (ii) calculating a ratio ((B)/(A)) of the amount (B) of tryptophan, kynurenine, or 3-hydroxyanthranilic acid to the amount (A) of anthranilic acid.

8. The method for obtaining data according to claim 7,
wherein the step 2 is a step of (i) calculating a ratio ((B)/(A)) of the amount (A) of anthranilic acid to the amount (B) of tryptophan, kynurenine, or 3-hydroxyanthranilic acid.

9. The method for obtaining data according to claim 7,
wherein the step 2 is a step of calculating a ratio of the amount of anthranilic acid to the amount of kynurenine or 3-hydroxyanthranilic acid.

10. The method for obtaining data according to claim 7,
wherein the psychiatric disorder is depressive disorder, bipolar disorder, schizophrenia, or dementia.

11. The method for obtaining data according to claim 7,
wherein the psychiatric disorder is depressive disorder.

12. The method for obtaining data according to claim 7,
wherein the sample is serum, plasma, whole blood, urine, or cerebrospinal fluid.

13. A marker for determining a psychiatric disorder comprising:
anthranilic acid; and
tryptophan, kynurenine, or 3-hydroxyanthranilic acid.
